# EUROPEAN PATENT APPLICATION

(11) **EP 1 553 090 A1**
(43) Date of publication of application: **13.07.2005**
(21) Application number: 03736158.1
(22) Date of filing: 11.06.2003
(51) Int. Cl.: C07D 239/47, C07D 263/42

(54) **PROCESS FOR PRODUCING PYRIMIDINE COMPOUND**

(30) Priority: 14.06.2002 JP 2002174916
(71) Applicant: Ajinomoto Co., Inc., Tokyo 104-8315 (JP)
(72) Inventor: TAKAHASHI, Daisuke, c/o AJINOMOTO CO., INC., Kawasaki-shi, Kanagawa 210-8681 (JP); HONDA, Yutaka, c/o AJINOMOTO CO., INC., Kawasaki-shi, Kanagawa 210-8681 (JP); IZAWA, Kunisuke, c/o AJINOMOTO CO., INC., Kawasaki-shi, Kanagawa 210-8681 (JP)
(74) Representative: Nicholls, Kathryn Margaret
(86) International application number: PCT/JP2003/007399
(87) International publication number: WO 2003/106434

(57) **Abstract**

Azlactone Compound (2) is reacted with Amidine Compound (3) to give Pyrimidine Compound (1) useful as an intermediate for enzyme inhibitors (e.g., elastase inhibitor, chymase inhibitor etc.): 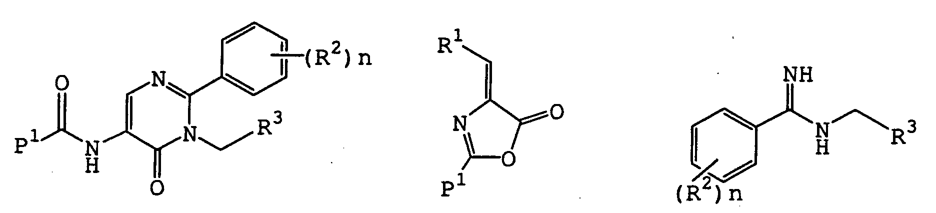 Pyrimidine Compound (1) Azlactone Compound (2) Amidine Compound (3) wherein each symbol is as defined in the specification.

## Description

### Technical Field

The present invention relates to a compound useful as an intermediate for pharmaceuticals such as enzyme inhibitors (e.g., elastase inhibitor and chymase inhibitor), and a method of preparing the same.

### Background Art

Various methods of preparing enzyme inhibitors having a 5-amino-6-oxo-2-phenylpyrimidine backbone, such as elastase inhibitors and chymase inhibitor, have been reported. For example, in WO93/21210 and WO93/21214, a method of preparing 2-(5-(benzyloxycarbonyl)amino-6-oxo-2-phenyl-1,6-dihydropyrimidin-1-yl)acetic acid, which is an intermediate for enzyme inhibitors having a 5-amino-6-oxo-2-phenylpyrimidine backbone, from cyanobenzene per the following scheme is disclosed. However, because this method involves a large number of steps and uses an expensive reagent, and also because a risk of explosion associated with Crutius rearrangement is of concern when the method is conducted on an industrial scale, the method cannot be said to be an always industrially suitable method. 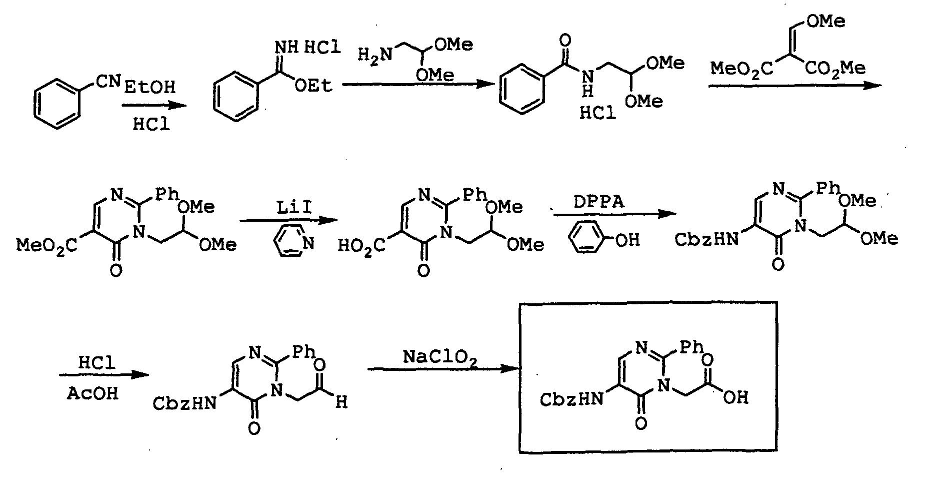 (In the scheme, Cbz represents a benzyloxycarbonyl group, DPPA represents diphenylphosphorylazide, and Ac represents an acetyl group.)

Also, in WO01/23361, a method of preparing a 5-amino-1-(substituted)-carbonylmethyl-2-phenyl-6-oxopyrimidine compound, which is an elastase inhibitor, is disclosed. The production involves 2-(5-nitro-6-oxo-2-phenyl-1,6-dihydropyrimidin-1-yl)acetic acid as an intermediate, and a method of preparing this acetic acid compound from a nitroacetic acid ester per the following scheme is disclosed. Although this method is preferable in that the number of steps is smaller than foregoing WO93/21210 and WO93/21214, it cannot be said to be an always industrially suitable method because the nitroacetic acid ester is difficult to obtain industrially, and also because a risk of explosion is of concern because of its identity as a nitro compound.

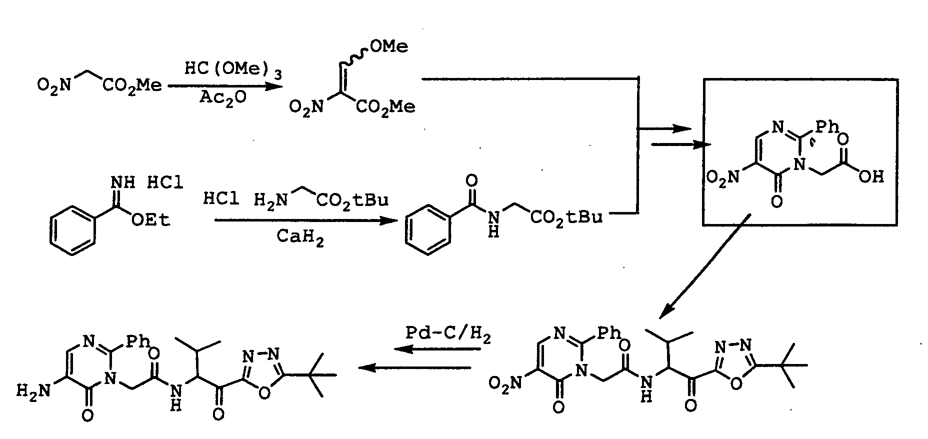

### (In the scheme, Ac represents an acetyl group.)

As described above, there are problems in the conventional methods of preparing compounds used as enzyme inhibitor intermediates. Hence, there is a need for the development of an enzyme inhibitor intermediate that can be prepared safely with a small number of steps at a relatively low cost.

The object of the present invention is to provide an enzyme inhibitor intermediate that can be prepared safely with a small number of steps at a relatively low cost and a method of preparing the same.

### Disclosure of the Invention

The present inventors, as a result of an extensive investigation with the aim of solving the above-described problems, found that a novel compound having a 5-amino-4-oxopyrimidine backbone, represented by the formula (1) below, can be used as an enzyme inhibitor intermediate, further found a method by which this intermediate can be prepared safely with a small number of steps at a relatively low cost, and developed the present invention.

Also, the acyl group (particularly the benzoyl group) and the like which are present in the pyrimidine compound represented by the formula (1) below are difficult to deprotect; in case of deprotection by a conventional method (in the presence of a strongly acidic or strongly basic compound), it had been difficult to obtain a deprotected derivative with a good yield due to the accompanying decomposition of the pyrimidine compound itself and side reactions. Thus, the present inventors conducted an extensive investigation of deprotection conditions, as a result, surprisingly succeeded in suppressing the decomposition and side reactions which had been problematic, by conducting the deprotection reaction in an alcohol in the presence of an alkali metal hydroxide, in addition found that by obtaining a deprotected derivative (that is, an acetic acid compound represented by the formula (6) below) as a direct salt, the deprotected derivative can easily be separated and purified from the compound prepared in the deprotection reaction (corresponding to benzoic acid when the amino-protecting group is a benzoyl group), and developed the present invention.

Furthermore, the present inventors found that by utilizing the above-described method of preparing a pyrimidine compound and an acetic acid compound, an N-protected compound represented by the formula (7) below, wherein the amino group of the acetic acid compound is protected, can be prepared safely at a relatively low cost with a good yield.

That is, the present invention is as follows.
[1] A method of preparing a pyrimidine compound represented by the formula (1) : 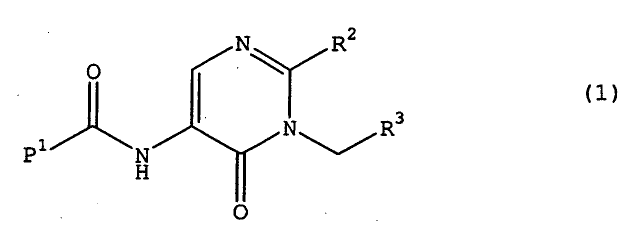 wherein P¹ represents a hydrogen atom, an alkyl group, an optionally substituted aryl group, an alkenyl group, an aralkyl group, a haloalkyl group or an optionally substituted amino group, R² represents an alkyl group having 1 to 4 carbon atoms or a phenyl group optionally substituted by at least one substituent selected from among an optionally substituted alkyl group, an alkoxy group, a nitro group, a hydroxy group, a halogen atom and an amino group and R³ represents a carboxyl group or a group that can be derivatized to a carboxyl group, provided that when P¹ is a phenyl group, then R³ should represent a group that can be derivatized to a carboxyl group, or a salt thereof (hereinafter to be referred to as Pyrimidine Compound (1) unless otherwise specified), which comprises reacting an azlactone compound represented by the formula (2): 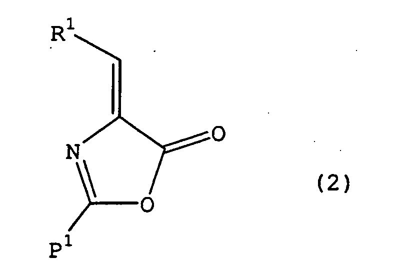 wherein R¹ represents an alkoxy group or a trialkylsiloxy group and P¹ is as defined above, or a salt thereof (hereinafter to be referred to as Azlactone Compound (2) unless otherwise specified), with an amidine compound represented by the formula (3): 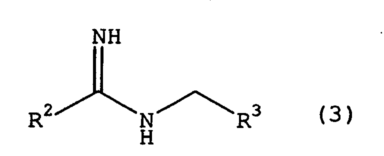 wherein R² is as defined above, or a salt thereof (hereinafter referred to as Amidine Compound (3) unless otherwise specified).
[2] The production method of [1] above, wherein an acid adduct salt of Amidine Compound (3) is neutralized with a base and thereafter reacted with Azlactone Compound (2).
[3] The production method of [1] above, wherein the Azlactone Compound (2) obtained by reacting an α-aminocarboxylic acid represented by the formula (4): 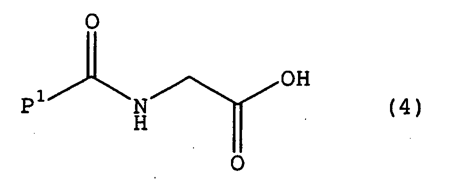 wherein P¹ is as defined in [1] above, or a salt thereof (hereinafter referred to as α-Aminocarboxylic Acid (4) unless otherwise specified), with an ortho-formic acid ester represented by the formula (5): (R¹)₃CH wherein R¹ is as defined in [1] above (hereinafter referred to as Ortho-Formic Acid Ester (5)) is used.
[4] The production method of any of [1] to [3] above, wherein P¹ is an alkyl group, an optionally substituted aryl group, an alkenyl group, an aralkyl group, a haloalkyl group or an optionally substituted amino group.
[5] The production method of any of [1] to [4] above, wherein P¹ is a chlorophenyl group, a tolyl group, a phenyl group, a benzyl group or a methyl group,
   R¹ is a methoxy group or an ethoxy group,
   R³ is a tert-butoxycarbonyl group or a carboxyl group, and
   R² is a methyl group, a phenyl group or a fluorophenyl group.
[6] The production method of any of [1] to [4] above, wherein P¹ is a phenyl group, a benzyl group or a methyl group, R¹ is a methoxy group or an ethoxy group, R³ is a tert-butoxycarbonyl group, and R² is a methyl group, a phenyl group or a fluorophenyl group.
[7] A method or preparing an acetic acid compound represented by the formula (6): 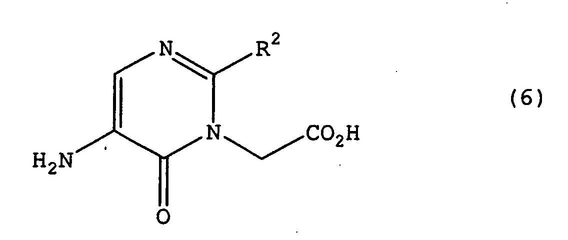 wherein R² is as defined in [1] above, or a salt thereof (hereinafter referred to as Acetic Acid Compound (6) unless otherwise specified), which comprises reacting Azlactone Compound (2) with Amidine Compound (3) to yield Pyrimidine Compound (1), and subjecting the obtained Pyrimidine Compound (1) to a deprotection reaction, wherein when P¹ is a phenyl group, then R³ should be a group that can be derivatized to a carboxyl group.
[8] The production method of [7] above, wherein P¹ is an alkyl group, an optionally substituted aryl group, an alkenyl group, an aralkyl group, a haloalkyl group or an optionally substituted amino group.
[9] A method of preparing Acetic Acid Compound (6), which comprises subjecting Pyrimidine Compound (1) to a deprotection reaction, wherein when P¹ is a phenyl group, then R³ should be a group that can be derivatized to a carboxyl group.
[10] The production method of [9] above, wherein P¹ is an alkyl group, an optionally substituted aryl group, an alkenyl group, an aralkyl group, a haloalkyl group or an optionally substituted amino group.
[11] The production method of [7], [9] or [10] above, wherein Pyrimidine Compound (1) is subjected to a deprotection reaction in an alcohol in the presence of an alkali metal hydroxide.
[12] The production method of [10] above, wherein P¹ is a phenyl group.
[13] A method of preparing an N-protected compound represented by the formula (7): 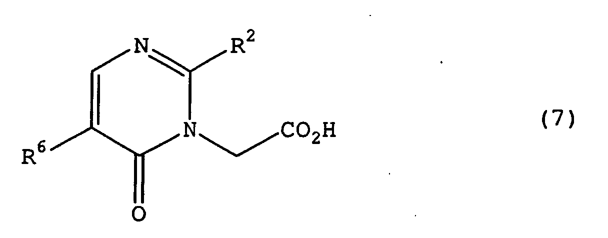 wherein R² represents an alkyl group having 1-4 carbon atoms or a phenyl group optionally substituted by at least one substituent selected from among an optionally substituted alkyl group, an alkoxy group, a nitro group, a hydroxy group, a halogen atom and an amino group, and R⁶ represents an -NR⁵H group or an -N(R⁵)₂ group wherein R⁵ represents an amino-protecting group, or a salt thereof (hereinafter referred to as N-Protected Compound (7) unless otherwise specified), which comprises the following steps 1 to 3;
   step 1: reacting Azlactone Compound (2) with Amidine Compound (3) to yield Pyrimidine Compound (1),
   step 2: subjecting Pyrimidine Compound (1) to a deprotection reaction to yield Acetic Acid Compound (6),
   step 3: subjecting Acetic Acid Compound (6) to a reaction for protecting the amino group substituted at the 5-position of the pyrimidine ring to yield N-Protected Compound (7);
   provided that when P¹ is a phenyl group, then R³ should be a group that can be derivatized to a carboxyl group.
[14] The production method of [13] above, wherein P¹ is an alkyl group, an optionally substituted aryl group, an alkenyl group, an aralkyl group, a haloalkyl group or an optionally substituted amino group.
[15] The production method of [13] or [14] above, wherein step 2 is conducted in an alcohol in the presence of an alkali metal hydroxide.
[16] A method of preparing an N-protected compound represented by the formula (7'): 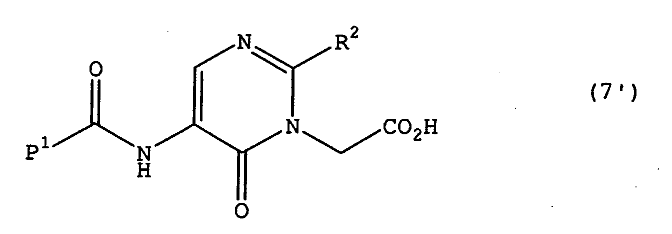 wherein P¹ represents a hydrogen atom, an alkyl group, an optionally substituted aryl group, an alkenyl group, an aralkyl group, a haloalkyl group or an optionally substituted amino group, and R² represents an alkyl group having 1 to 4 carbon atoms or a phenyl group optionally substituted by at least one substituent selected from among an optionally substituted alkyl group, an alkoxy group, a nitro group, a hydroxy group, a halogen atom and an amino group, or a salt thereof (hereinafter referred to as N-Protected Compound (7') unless otherwise specified), which comprises derivatizing R³' in a pyrimidine compound represented by the formula (1'): 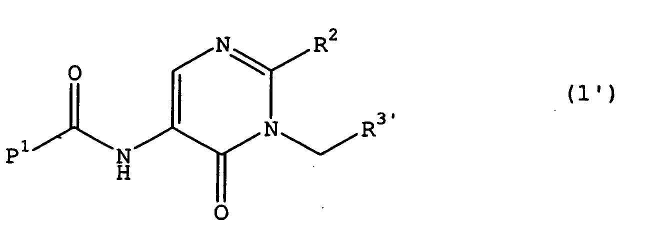 wherein P¹ and R² are as defined above and R³' represents a group that can be derivatized to a carboxyl group, or a salt thereof (hereinafter referred to as Pyrimidine Compound (1') unless otherwise specified) to a carboxyl group.
[17] The production method of [16] above, wherein P¹ is an alkyl group, an optionally substituted aryl group, an alkenyl group, an aralkyl group, a haloalkyl group or an optionally substituted amino group.
[18] Pyrimidine Compound (1).
[19] The pyrimidine compound of [18] above, wherein P¹ is an alkyl group, an optionally substituted aryl group, an alkenyl group, an aralkyl group, a haloalkyl group or an optionally substituted amino group, or a salt thereof.
[20] The pyrimidine compound of [19] above, wherein P¹ represents an alkyl group, an aryl group or an aralkyl group, R² represents a phenyl group optionally substituted by a halogen atom or an alkyl group having 1-4 carbon atoms, and R³ represents a carboxyl group or a group that can be derivatized to a carboxyl group.
[21] A method of preparing Azlactone Compound (2), which comprises reacting α-Aminocarboxylic Acid (4) with Ortho-Formic Acid Ester (5) in the presence of acetic anhydride, zinc chloride and sodium acetate.
[22] An azlactone compound represented by the formula (2'): 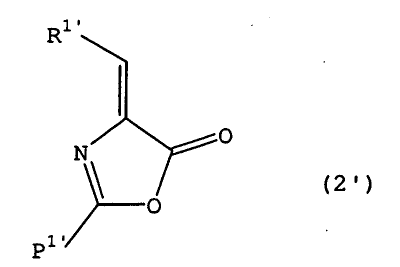 wherein P¹' represents an alkyl group, and R¹' represents an alkoxy group.
[23] The azlactone compound of [22] above, wherein P¹' is a methyl group, an ethyl group, an isopropyl group, a tert-butyl group or an n-propyl group, and R¹' is a methoxy group or an ethoxy group.
[24] A method of preparing the azlactone compound of [22] above or a salt thereof, which comprises reacting an α-aminocarboxylic acid represented by the formula (4'): 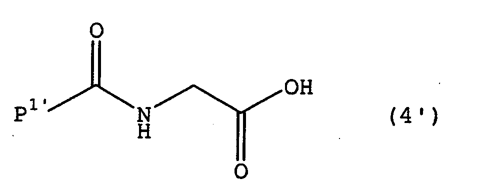
wherein P¹' represents an alkyl group, or a salt thereof with an ortho-formic acid ester represented by the formula (5'): (R¹')₃CH wherein R¹' represents an alkoxy group.

### Mode of Embodiment of the Invention

The alkyl group for P¹ or P¹' is a linear or branched alkyl having preferably 1-20, more preferably 1-7, carbon atoms; for example, a methyl group, an ethyl group, an n-propyl group, an isopropyl group, an n-butyl group, an isobutyl group, a sec-butyl group, a tert-butyl group, a lauryl group and the like can be mentioned. Particularly preferred are a methyl group and an ethyl group.

The optionally substituted aryl group for P¹ is an aryl group having preferably 6-20, more preferably 6-8, carbon atoms, and the aryl group is optionally substituted by 1 or more of the following substituents. As examples of the substituents mentioned here, a nitro group, a linear or branched alkoxy group (number of carbon atoms: 1-6, e.g., methoxy group), a halogen atom (e.g., chlorine atom, fluorine atom and the like), a linear or branched alkyl group (preferred number of carbon atoms: 1-4, e.g., methyl group, ethyl group, propyl group and the like) and the like can be mentioned. As specific examples of the optionally substituted aryl group, a phenyl group, a nitrophenyl group, a methoxyphenyl group, a chlorophenyl group, a fluorophenyl group, a tolyl group and the like can be mentioned; particularly preferred is a phenyl group.

The aralkyl group for P¹ represents an aralkyl group wherein the aryl moiety is an aryl group having preferably 6-12, more preferably 6-8, carbon atoms, and the alkyl moiety is a linear or branched alkyl group having preferably 1-6, more preferably 1-3, carbon atoms. As specific examples of the aralkyl group, a benzyl group is preferred.

The alkenyl group for P¹ is a linear or branched alkenyl group having preferably 2-20, more preferably 2-7, carbon atoms; for example, a vinyl group, an allyl group, a homoallyl group, an oleyl group and the like can be mentioned; particularly preferred are a vinyl group and an allyl group.

The haloalkyl group for P¹ is a linear or branched alkyl group having preferably 1-5, more preferably 1-3, carbon atoms, substituted by 1 or 2 or more halogen atoms (fluorine atom, chlorine atom, bromine atom, iodine atom); for example, a trifluoromethyl group, a trichloromethyl group, a chloromethyl group and the like can be mentioned; a trifluoromethyl group is preferred.

The optionally substituted amino group for P¹ is an amino group optionally substituted by 1 or 2 of the following substituents; in the case of di-substitution, the individual substituents may be identical or different. As the substituents mentioned here, a linear or branched alkyl group (number of carbon atoms: 1-6, e.g., methyl, ethyl, isopropyl) and the like can be mentioned. As specific examples of the optionally substituted amino group, an amino group, a methylamino group, an ethylamino group, an isopropylamino group and the like can be mentioned; particularly preferred is an amino group.

The alkoxy group for R¹ or R¹' is a linear or branched alkoxy group having preferably 1-20, more preferably 1-10, carbon atoms; for example, a methoxy group, an ethoxy group, an n-propyloxy group, an isopropyloxy group, an n-butyloxy group, an isobutyloxy group, a tert-butoxy group and the like can be mentioned. Particularly preferred are a methoxy group and an ethoxy group.

The trialkylsiloxy group for R¹ is a siloxy group trisubstituted by identical or different alkyl groups, and each alkyl group is a linear or branched alkyl group having preferably 1-3 carbon atoms. As specific examples of the trialkylsiloxy group, a trimethylsiloxy group, a triethylsiloxy group and the like can be mentioned; particularly preferred is a trimethylsiloxy group.

The alkyl group having 1-4 carbon atoms for R² is linear or branched; for example, methyl, ethyl, propyl, isopropyl, n-butyl, isobutyl, sec-butyl, tert-butyl and the like can be mentioned.

The optionally substituted alkyl group which is one of the substituents for the phenyl group for R² is a linear or branched alkyl group having preferably 1-5, more preferably 1-3, carbon atoms, and the alkyl group is optionally substituted by 1 or more of the following substituents. As examples of the substituents, a hydroxy group, a nitro group and the like can be mentioned. As specific examples of the optionally substituted alkyl group, a methyl group, an ethyl group, an n-propyl group, an isopropyl group, an n-butyl group, an isobutyl group, a sec-butyl group, a tert-butyl group, a hydroxymethyl group, a nitromethyl group and the like can be mentioned; particularly preferred are a methyl group and an ethyl group.

The halogen atom as one of the substituents for the phenyl group for R² is a fluorine atom, a chlorine atom, a bromine atom or an iodine atom; particularly preferred are a fluorine atom and a chlorine atom.

The alkoxy group as one of the substituents for the phenyl group for R² is a linear or branched alkoxy group having preferably 1-6, more preferably 1-3, carbon atoms; for example, a methoxy group, an ethoxy group, an n-propyloxy group, an isopropyloxy group, an n-butyloxy group, an isobutyloxy group, a tert-butoxy group and the like can be mentioned. Particularly preferred is a methoxy group.

As the group that can be derivatized to a carboxyl group for R³ or R³', a nitrile group, a methylhydroxy group, a carbonylamide group, a formyl group, a vinyl group, a dimethoxymethyl group, a diethoxymethyl group, a group represented by General the formula (4): -COOR⁴ wherein R⁴ represents a hydrogen atom, an alkyl group, an optionally substituted aralkyl group, an optionally substituted aryl group, an alkali metal or an alkaline earth metal) and the like can be mentioned.

The alkyl group for R⁴ is a linear or branched alkyl group having preferably 1-20, more preferably 1-6, carbon atoms; for example, a methyl group, an ethyl group, an n-propyl group, an isopropyl group, an n-butyl group, an isobutyl group, a sec-butyl group, a tert-butyl group and the like can be mentioned. Particularly preferred is a tert-butyl group.

The optionally substituted aralkyl group for R⁴ is an aralkyl group having preferably 7-12 carbon atoms, and the aralkyl group is optionally substituted by 1 or more of the following substituents. As examples of the substituents, a nitro group, a linear or branched alkoxy group (number of carbon atoms: 1-3, e.g., methoxy group) and the like can be mentioned. As specific examples of the optionally substituted aralkyl group, a benzyl group, a nitrobenzyl group, a methoxybenzyl group and the like can be mentioned; particularly preferred is a benzyl group.

The optionally substituted aryl group for R⁴ is an aryl group having preferably 6-10 carbon atoms, and the aryl group is optionally substituted by 1 or more of the following substituents. As examples of the substituents, a nitro group, a linear or branched alkoxy group (number of carbon atoms: 1-3, e.g., methoxy group) and the like can be mentioned. As specific examples of the optionally substituted aryl group, a phenyl group, a nitrophenyl group, a methoxyphenyl group and the like can be mentioned; particularly preferred is a phenyl group.

As the alkali metal for R⁴, sodium, potassium, lithium and the like are preferable.

As the alkaline earth metal for R⁴, 1/2 calcium and 1/2 magnesium are preferable.

As examples of the amino-protecting group for R⁵, an aralkyloxycarbonyl group (total number of carbon atoms: 8-13, e.g., benzoxycarbonyl group), an alkyloxycarbonyl group (total number of carbon atoms: 2-20, e.g., methyloxycarbonyl group, tert-butoxycarbonyl group), a phthaloyl group, a benzylidene group, a monobenzyl group, a dibenzyl group, a formyl group, an acetyl group and the like can be mentioned; particularly preferred is a benzyloxycarbonyl group.

P¹ is preferably an alkyl group, an optionally substituted aryl group or aralkyl group, or an alkyl group, an aryl group or an aralkyl group,
R² is preferably a phenyl group or an alkyl group optionally substituted by a halogen atom,
R³ is preferably a carboxyl group or a group that can be derivatized to a carboxyl group.

As R², a non-substituted phenyl group is preferred.

The compounds represented by the formulas (1)-(4), (6), (7), (1'), (2') and (7'), when having an acidic group, are capable of forming an alkali metal salt (for example, potassium salt, sodium salt, lithium salt and the like), an organic amine salt (for example, triethylamine salt, dicyclohexylamine salt and the like) and the like, and, when having a basic group, are capable of forming salts such as an inorganic acid salt (for example, hydrochloride, sulfate and the like) and an organic acid salt (for example, acetate, trifluoroacetate, tosylate, mesylate and the like).

### Method of Producing Pyrimidine Compound (1)

Conventional enzyme inhibitor intermediates cannot be prepared safely with a small number of steps at a relatively low cost, which in turn had been a cause of enzyme inhibitor yield reductions and the like. In contrast, novel Pyrimidine Compound (1) in the present invention can be prepared safely with a small number of steps at a relatively low cost, and is a compound that is useful as an enzyme inhibitor intermediate. Referring to Pyrimidine Compound (1), a case wherein P¹ represents an alkyl group, an optionally substituted aryl group or aralkyl group, or an alkyl group, an aryl group or an aralkyl group, R² represents a phenyl group optionally substituted by a halogen atom or an alkyl group having 1-4 carbon atoms, and R³ represents a carboxyl group or a group that can be derivatized to a carboxyl group, is preferred.

Pyrimidine Compound (1) can be obtained by reacting Azlactone Compound (2) with Amidine Compound (3). This reaction is conducted in a solvent; specifically, for example, Azlactone Compound (2) or a solution thereof is added to Amidine Compound (3) or a solution thereof (preferably added drop by drop, in the case of a solution), and the mixed solution is heated and stirred. The sequence of additions may be reverse to this.

Amidine Compound (3) tends to be decomposed in the free form and, as a solid, is normally isolated in the form of a stable salt. As examples of the salt of Amidine Compound (3), acid adduct salts such as hydrochloride, sulfate and ', trifluoroacetate can be mentioned. Note that Amidine Compound (3), when R³ is a carboxyl group, occurs stably in the form of a salt with the imino group in the molecule, and also occurs stable as a base adduct salt such as sodium salt or potassium salt. Therefore, when Amidine Compound (3), which is the free form, is reacted with Azlactone Compound (2), it is preferable to use a base (for example, alkoxide compounds such as sodium ethoxide, potassium butoxide and sodium methoxide) in an amount necessary to convert Amidine Compound (3) to a base adduct salt.

In the present invention, these acid adduct salts of Amidine Compound (3) are preferably reacted with Azlactone Compound (2) after being neutralized in a solvent using a base and once converted to the free form. The reaction can also be conducted by, for example, dissolving a salt of Amidine Compound (3) and Azlactone Compound (2) in a solvent, and adding a base, without previously conversion to the free form before the reaction; in this case, however, the yield of Pyrimidine Compound (1), which is the desired product, decreases. The base used for neutralization is not subject to limitation; for example, sodium hydroxide, potassium hydroxide, sodium carbonate, potassium carbonate, triethylamine, sodium methoxide, sodium ethoxide and the like can be mentioned. These bases may be used as aqueous solutions. Neutralization is normally conducted in a solvent; as the solvent, the same solvent as that used for the reaction with Azlactone Compound (2) can be used. Although the amount of base used is not subject to limitation, as long as it is an amount sufficient to convert the salt of Amidine Compound (3) to the free form, it is preferable from an economic idea that the amount be 3 equivalents or less, relative to Amidine Compound (3).

As the solvent usable for the reaction of Azlactone Compound (2) and Amidine Compound (3), any solvent can be used, as long as it does not interfere with the present reaction; for example, acetic acid esters (for example, ethyl acetate, ', isopropyl acetate, isobutyl acetate, n-butyl acetate and the like), hydrocarbons (for example, toluene, benzene, xylene and the like), acetonitrile, tetrahydrofuran (THF), alcohols (for example, ethanol, isopropyl alcohol, n-butanol and the like), dimethylformamide and the like can be mentioned, and these may be used alone or in combination of 2 kinds or more; acetonitrile, toluene, ethanol and ethyl acetate are preferable, acetonitrile, toluene and isopropyl alcohol, used alone or in a mixed solvent, are more preferable; particularly preferred are acetonitrile and toluene, used alone or in a mixed solvent. The amount of solvent used is normally 3-100 times by weight, preferably 5-50 times by weight, relative to Azlactone Compound (2).

The amount of Amidine Compound (3) used is normally 1-5 equivalents, preferably 1-2 equivalents, relative to Azlactone Compound (2).

The reaction of Azlactone Compound (2) and Amidine Compound (3) is normally conducted from 20°C to the range of the reflux temperature of the solvent used (preferably 30-90°C). The reaction, in the above-described temperature range, normally completes in 1 hour to 48 hours (preferably 3 hours to 24 hours).

Isolation and purification of Pyrimidine Compound (1) can be conducted by a conventional method. For example, after completion of the reaction, by sequentially washing the reaction mixture with an acidic aqueous solution (for example, hydrochloric acid, sulfuric acid and the like) and an alkali aqueous solution (for example, saturated aqueous sodium bicarbonate, saline and the like), separating the liquid, and concentrating the obtained organic layer, Pyrimidine Compound (1) can be purified. Furthermore, by adding to this a crystallization solvent (for example, ethers (e.g., diethyl ether, THF and the like), acetone, acetonitrile, hydrocarbon-series solvents (for example, toluene, benzene, hexane, heptane and the like), halogen-series solvents (for example, dichloromethane, dichloroethane and the like), alcohols (for example, methanol, ethanol, isopropanol and the like), water or mixed solvents thereof and the like) to cause crystallization, Pyrimidine Compound (1) can be isolated.

Azlactone Compound (2) and Amidine Compound (3) used for the production of Pyrimidine Compound (1) can be prepared by a known method. Amidine Compound (3) can be obtained by, for example, a method described in WO93/21210 and WO93/21214 or a method based thereon. Azlactone Compound (2) can be prepared by a known production method; particularly preferred from the viewpoint of economy and efficiency is production by the method described in detail below.

### Method of preparing Azlactone Compound (2)

Azlactone Compound (2) can be prepared by reacting α-Aminocarboxylic Acid (4) with Ortho-Formic Acid Ester (5). It is preferable that this reaction be conducted in the presence of acetic anhydride. Also, although this reaction can be conducted without a solvent, it can also be conducted in a solvent. Specifically, for example, α-Aminocarboxylic Acid (4), along with Ortho-Formic Acid Ester (5) and acetic anhydride, is heated and stirred without a solvent or in a solvent. The sequence of additions of the reagents is not limited to this, and can be appropriately changed.

As the solvent usable for the reaction of α-Aminocarboxylic Acid (4) and Ortho-Formic Acid Ester (5), any solvent can be used, as long as it does not interfere with this reaction; for example, acetic acid esters (for example, ethyl acetate, methyl acetate, isopropyl acetate, n-butyl acetate and the like), toluene, acetonitrile, acetic acid, THF and the like can be mentioned; preferred are ethyl acetate and toluene. The amount of solvent used is normally 1-20 times by weight, preferably 1-10 times by weight, relative to α-Aminocarboxylic Acid (4).

The amount of Ortho-Formic Acid Ester (5) used is normally 1-5 equivalents, preferably 1-3 equivalents, relative to α-Aminocarboxylic Acid (4).

The amount of acetic anhydride used is normally 3-20 equivalents, preferably 3-10 equivalents, relative to α-Aminocarboxylic Acid (4).

The reaction of α-Aminocarboxylic Acid (4) and Ortho-Formic Acid Ester (5) is normally conducted from 40°C to the range of the reflux temperature of the solvent used (preferably 50-100°C). The reaction, in the above-described temperature range, normally completes in 30 minutes to one night (preferably 1 hour to 3 hours).

The reaction of α-Aminocarboxylic Acid (4) and Ortho-Formic Acid Ester (5) is preferably conducted by using zinc chloride and sodium acetate, in addition to acetic anhydride, because Azlactone Compound (2) can be obtained with a better yield. The sequence of their additions is not subject to limitation; for example, Ortho-Formic Acid Ester (5), acetic anhydride, zinc chloride and sodium acetate are added to α-Aminocarboxylic Acid (4) at one time.

The amount of zinc chloride used for the reaction of α-Aminocarboxylic Acid (4) and Ortho-Formic Acid Ester (5) is normally 0.001-1.0 mol equivalents, preferably 0.01-0.2 mol equivalents, relative to α-Aminocarboxylic Acid (4).

The amount of sodium acetate used is normally 0.001-1.0 mol equivalents, preferably 0.01-0.2 mol equivalents, relative to α-Aminocarboxylic Acid (4).

When acetic anhydride, zinc chloride and sodium acetate are used, the reaction of α-Aminocarboxylic Acid (4) and Ortho-Formic Acid Ester (5) is normally conducted in the range of 50-110°C (preferably 60-90°C). The reaction, in the above-described temperature range, normally completes in 10 minutes to 5 hours (preferably 30 minutes to 3 hours).

Isolation and purification of Azlactone Compound (2) can be conducted by a conventional method. For example, by cooling the obtained reaction liquor, adding an organic solvent (for example, toluene, alcohol (for example, ethanol, methanol, isopropanol and the like) and the like), concentrating the liquor, and crystallizing this by the addition of a crystallization solvent (for example, hexane, heptane, water and the like) or subjecting this to silica gel column chromatography, Azlactone Compound (2) can be isolated.

Referring to the Azlactone Compound (2) obtained by the above-described method, a compound wherein P¹ is an alkyl group (e.g., methyl group, ethyl group, isopropyl group, tert-butyl group, n-propyl group and the like) and R¹ is an alkoxy group (e.g., methoxy group, ethoxy group and the like) (that is, Compound (2')) is novel.

α-Aminocarboxylic Acid (4) and Ortho-Formic Acid Ester (5) used for the production of Azlactone Compound (2) are each easy to obtain industrially, or can be prepared by an ordinary acylation reaction.

Also by subjecting the obtained Azlactone Compound (2) as is, without isolation, to the reaction with Amidine Compound (3), Pyrimidine Compound (1) can be obtained. When Azlactone Compound (2) as is, without isolation, is subjected to the reaction with Amidine Compound (3), the sample used for the reaction with Amidine Compound (3) can be used in the above-described range with α-Aminocarboxylic Acid (4), instead of Azlactone Compound (2), as the index.

### Method of preparing Acetic Acid Compound (6)

Pyrimidine Compound (1) has a protected amino group at the 5-position of the pyrimidine ring. Furthermore, when R³ is a group that can be derivatized to a carboxyl group, the substituent at the 1-position of the pyrimidine ring has a protected carboxyl group. That is, the deprotection reaction of Pyrimidine Compound (1) in the present invention means a reaction which removes not only the amino-protecting group, but also the protecting group for the carboxyl group, when R³ is a group that can be derivatized to a carboxyl group.

The amino-protecting group (for example, acyl group (particularly benzoyl group)) present in Pyrimidine Compound (1) is difficult to remove, and normally needs to be removed in the presence of a strongly acidic or strongly basic compound. The present inventors conducted deprotection of Pyrimidine Compound (1) under some conditions; as a result (see the Reference Example given below), it was difficult to obtain the deprotected derivative at a good yield due to the accompanying decomposition of the pyrimidine compound itself and side reactions.

Thus, the present inventors conducted an extensive investigation of deprotection conditions with the aim of solving these problems. As a result, the present inventors surprisingly succeeded in suppressing the decomposition and side reactions which had been problematic, by conducting the deprotection in an alcohol in the presence of an alkali metal hydroxide, and in addition found that by obtaining a deprotected derivative as a direct salt, the deprotected derivative can easily be separated and purified from the compound prepared in the deprotection reaction (for example, corresponding to benzoic acid when the amino-protecting group is a benzoyl group).

That is, it is preferable to subject Pyrimidine Compound (1) to a deprotection reaction in an alcohol in the presence of an alkali metal hydroxide, because the decomposition of the pyrimidine compound itself and the occurrence of side reactions can be suppressed and Acetic Acid Compound (6) can be prepared with a good yield. The method of preparing Acetic Acid Compound (6) is hereinafter described with reference to the preferred mode of embodiment but the production method of the present invention is not by any means limited to this mode of embodiment.

Specifically, Pyrimidine Compound (1), an alkali metal hydroxide and an alcohol are heated and stirred. The temperature and reaction time for this operation are not subject to limitation; the reaction normally completes, from 60°C to the range of the reflux temperature of the solvent used, in 3-20 hours.

As the alcohol used for the deprotection reaction, an alcohol having preferably 1-4 carbon atoms can be mentioned; for example, methanol, ethanol, isopropyl alcohol, butanol and the like can be mentioned; particularly preferred is methanol. The amount of alcohol used is normally 5-20 times by weight, preferably 8-15 times by weight, relative to Pyrimidine Compound (1).

As examples of alkali metal hydroxides usable for the deprotection reaction, lithium hydroxide, sodium hydroxide, potassium hydroxide and the like can be mentioned; sodium hydroxide is preferred. These may be used alone or in combination of 2 kinds or more, and the alkali metal hydroxide can also be used as a hydrate. The total amount of alkali metal hydroxide used is normally 1-20 equivalents, preferably 1-10 equivalents, relative to Pyrimidine Compound (1).

As an example of a method of preparing Acetic Acid Compound (6) other than the above-described preferred mode of embodiment, a method wherein deprotection of Pyrimidine Compound (1) is conducted in a solvent like acetonitrile, water, ethanol or acetic acid (0.5-20 times by weight, relative to Pyrimidine Compound (1)) in the presence of an acid such as hydrochloric acid, sulfuric acid or bromic acid (0.5-20 equivalents, relative to Pyrimidine Compound (1)) can be mentioned.

As the deprotection reaction proceeds, Acetic Acid Compound (6) precipitates as a crystal in the form of a salt (an alkali metal salt such as sodium salt or lithium salt). By cooling the reaction solution, and separating the resulting crystal by filtration and the like where necessary, a crystal of a salt of Acetic Acid Compound (6) can be obtained. Also, by neutralizing the reaction solution or the obtained crystal of the salt in a solvent with an acid (for example, hydrochloric acid), the salt of Acetic Acid Compound (6) can be converted to the free form. In this operation, the pH of the solution containing the salt of Acetic Acid Compound (6) is preferably adjusted to 0-3.5. The free form of Acetic Acid Compound (6) can be isolated as a crystal by a conventional method such as extraction or crystallization.

### Method of preparing N-Protected Compound (7)

N-Protected Compound (7) can be prepared by subjecting Acetic Acid Compound (6) to a reaction for protecting the amino group substituting at the 5-position of the pyrimidine ring. This reaction can normally be conducted using a reagent used in protecting the amino group (hereinafter referred to as amino-protecting reagent), for example, benzyloxycarbonyl chloride, methyloxycarbonyl chloride, tert-butoxycarbonyl chloride, phthaloylic anhydride, di-tert-butylcarbonate, benzaldehyde, monobenzyl bromide, monobenzyl chloride, acetic anhydride, formic acid/acetic anhydride and the like.

For example, when benzyloxycarbonyl chloride is the amino-protecting reagent, specifically, in a solvent, an amino-protecting reagent is added to Acetic Acid Compound (6), and stirring is conducted. In this operation, the pH of the reaction system is preferably adjusted to preferably 4-11, more preferably 5-8, and the adjustment of the pH can be conducted by the addition of a base (for example, sodium hydroxide, sodium hydrogen carbonate and the like).

As the solvent usable for the reaction of Acetic Acid Compound (6) and an amino-protecting reagent, any solvent can be used, as long as it does not interfere with this reaction; for example, water, THF, an alcohol (for example, methanol and the like), acetone, acetonitrile and the like can be mentioned, and these can also be used as mixed solvents. Particularly preferred is water. The amount of solvent used is 5-20 times by weight, preferably 10-15 times by weight, relative to Acetic Acid Compound (6).

The amount of amino-protecting reagent used is normally 0.7-3 equivalents, preferably 0.7-1.5 equivalents, relative to Acetic Acid Compound (6).

The reaction of Acetic Acid Compound (6) and an amino-protecting reagent is normally conducted at 0-40°C (preferably 0-20°C). The reaction, in the above-described temperature range, normally completes in 1-20 hours (preferably 2-5 hours).

Isolation and purification of N-Protected Compound (7) can be conducted by a conventional method. For example, by acidifying (preferably to pH 0-3) the obtained reaction liquor by a means such as adding an acidic compound (for example, hydrochloric acid and the like) when a base, for example, is used for the reaction, extracting this by the addition of an organic solvent (for example, ethyl acetate and the like), concentrating the organic layer, and then adding a crystallization solvent (for example, hexane and the like) to cause crystallization, N-Protected Compound (7) can be isolated. To further increase the purity, the isolated N-Protected Compound (7) can also be subjected to recrystallization and the like.

Acetic Acid Compound (6), which is a raw material for N-Protected Compound (7), can be obtained by subjecting Pyrimidine Compound (1) to a deprotection reaction. After Pyrimidine Compound (1) is subjected to the deprotection reaction, N-Protected Compound (7) can be prepared as is, without isolating Acetic Acid Compound (6). In this case, the sample used in preparing N-Protected Compound (7) can be used in the above-described range with Pyrimidine Compound (1), instead of Acetic Acid Compound (6), as the index.

### Method of preparing N-Protected Compound (7')

N-Protected Compound (7') can be obtained by derivatizing R³ in Pyrimidine Compound (1') to a carboxyl group. In the present invention, "derivatizing to a carboxyl group" means that a group that can be derivatized to a carboxyl group in Pyrimidine Compound (1') is directly or indirectly derivatized to a carboxyl group. Indirectly derivatizing to a carboxyl group means that R³ is once derivatized to a group and then derivatized to a carboxyl group.

In the reaction for "derivatizing to a carboxyl group", the reaction needs to be conducted without removing the amino-protecting group substituting at the 1-position of the pyrimidine ring. The reaction for "derivatizing to a carboxyl group" varies depending on the kind of R³; for example, R³ can be derivatized to a carboxyl group by subjecting Pyrimidine Compound (1') to a reaction commonly used as a deprotection reaction for a carboxyl group or a reaction based thereon. Specific examples of the reaction for "derivatizing to a carboxyl group" are given below but the method of the present invention is not by any means limited to these specific examples.
(1) When R³ is a dimethoxymethyl group, Pyrimidine Compound (1') is treated with an acid to derivatize R³ to a formyl group, and this is further subjected to an oxidization reaction.
(2) When R³ is a methylhydroxy group, Pyrimidine Compound (1') is subjected to oxidation with 2,2,6,6-tetramethyl-1-piperidinyloxy (TEMPO), potassium permanganate-sulfuric acid, sodium chlorite and the like.
(3) When R³ is a tert-butoxycarbonyl group, Pyrimidine Compound (1') is treated with an acid such as trifluoroacetic acid (TFA) or hydrochloric acid.
(4) When R³ is a methoxycarbonyl group or an ethoxycarbonyl group, Pyrimidine Compound (1') is subjected to an ordinary saponification reaction.
(5) When R³ is a nitrile group, Pyrimidine Compound (1') is heated in a basic aqueous solution.

### (1)-(5) above can be conducted by methods known to those skilled in the art.

All of the Pyrimidine Compounds (1) and (1'), Acetic Acid Compound (6) and N-Protected Compounds (7) and (7') obtained by the above-described methods are compounds that are useful as intermediates for enzyme inhibitors such as elastase inhibitors and chymase inhibitors. There are various methods of derivatizing these compounds to enzyme inhibitors; for example, by subjecting N-Protected Compound (7) or (7') to a known method like those described in WO93/21200, WO93/21214 and the like, or a method based thereon, after Acetic Acid Compound (6) is obtained from Pyrimidine Compound (1) and N-Protected Compound (7) is obtained from Acetic Acid Compound (6), or after N-Protected Compound (7') is obtained from Pyrimidine Compound (1'), an enzyme inhibitor can be obtained. However, when R³ is not a carboxyl group, it is necessary to derivatize R³ to a carboxyl group by a method known to those skilled in the art, and then subject it to the aforementioned known method and the like.

### Examples

The present invention is hereinafter described in more detail by means of the following examples, by which, however, the present invention is not limited by any means.

### Example 1

Hippuric acid (5.0 g, 27.9 mmol), triethyl ortho-formate (5.4 g, 36.3 mmol) and acetic anhydride (8.6 g, 83.7 mmol) were stirred, and refluxed for 3 hours. Subsequently, the mixture was cooled, toluene (50 ml) and ethanol (4 ml) were added, and the mixture was concentrated. Further, toluene (30 ml) was added and the mixture was concentrated under reduced pressure, after which hexane (10 ml) was added and the residue was crystallized under ice cooling. The precipitate was filtered and washed with hexane, and the mother liquor was concentrated, after which hexane (2 ml) was added and the residue was crystallized in the same manner to yield a second crystal. The obtained crystals were combined and dried under reduced pressure to yield a 4-ethoxymethylene-2-phenyl-5-azlactone crystal (4.24 g, 19.5 mmol).
¹H-NMR(CDCl₃)δppm: 1.48-1.51(3H,t,J=7.1Hz), 4.42-4.47(2H,q,J=7.1Hz), 7.35(1H,s), 7.45-7.56(3H,m), 8.06-8.09(2H,m).

### Example 2

N-(tert-butoxycarbonylmethyl)phenylamidine hydrochloride (4.2 g, 15.6 mmol) was stirred in toluene (6 ml) and water (6 ml), and sodium carbonate (7 g) was added. After the organic layer was separated, the water layer was extracted by the addition of toluene (5 ml), and the organic layers were combined, washed with saline and dried with sodium sulfate. To the solution, a solution of 4-ethoxymethylene-2-phenyl-5-azlactone (2.60 g, 12.0 mmol) in acetonitrile (4 ml) was added drop by drop over 30 minutes, and this mixed solution was stirred at room temperature for 1 hour and at 80°C overnight. Subsequently, the mixed solution was washed with 1N aqueous hydrochloric acid, saturated aqueous sodium bicarbonate and saline, the organic layer was concentrated, hexane (12 ml) was added, and the residue was crystallized. After stirring at room temperature, the crystal was collected by filtration and dried to yield 1-(tert-butoxycarbonylmethyl)-6-oxo-2-phenyl-5-benzoylamino-1,6-dihydropyrimidine (3.90 g, 96 mmol).
¹H-NMR(DMSO-d₆)δppm: 1.31(9H,s), 4.57(2H,s), 7.51-7.65(8H,m), 7.97-8.00(2H,m), 8.79(1H,s), 9.58(1H,s).
MS(ESI)M/Z MH+406.2

### Example 3

To acetylglycine (0.2 g, 1.7 mmol), triethyl ortho-formate (0.76 g), acetic anhydride (1.74 g) and ethyl acetate (1.5 ml) were added, and the mixture was stirred at 95°C overnight, after which the reaction solution was washed with saline, and the organic layer was concentrated, after which toluene (3 ml) was added, and the mixture was concentrated to dryness. N-(tert-butoxycarbonylmethyl)phenylamidine hydrochloride (2.22 mmol) was stirred in toluene (6 ml) and water, and sodium carbonate (1.6 g) was added. After the organic layer was separated, the water layer was extracted by the addition of toluene (3 ml), and the organic layers were combined, washed with saline and dried with sodium sulfate. To the obtained solution, the previously obtained dry solid was added, and the mixture was stirred at 60°C overnight and 80°C overnight. The reaction solution was washed with 1N hydrochloric acid, saturated aqueous sodium hydrogen carbonate and saturated saline, and the organic layer was concentrated to dryness to yield 1-(tert-butyl) 2-(2-phenyl-5-acetylamino-1,6-dihydropyrimidin-3-yl)acetic acid.
¹H-NMR(DMSO-d₆)δppm: 1.36(9H,s), 2.15(3H,s), 4.52(2H,s), 7.46-7.56(5H,m), 8.84(1H,s), 9.62(1H,s).
MS(ESI)M/Z MH+344.2

### Example 4

To 1-(tert-butoxycarbonylmethyl)-6-oxo-2-phenyl-5-benzoylamino-1,6-dihydropyrimidine (500 mg, 1.2 mmol), methanol (6 ml) and 28% sodium methoxide (261 mg) were added, and the mixture was stirred at 70°C overnight, after which water (3 ml) and 4N sodium hydroxide (0.54 ml) were added, and the mixture was stirred at room temperature for 4 hours. Subsequently, the solution was concentrated under reduced pressure, ethyl acetate was added, and the mixture was adjusted to pH 1 with hydrochloric acid and twice washed. After the mixture was adjusted to pH 7 with sodium hydroxide, benzyloxycarbonyl chloride (0.17 ml, 0.9 mmol) was added gradually, and the mixture was stirred at room temperature for 2 hours while adjusting to pH 7. After washing with ethyl acetate, the solution was extracted by the addition of ethyl acetate and hydrochloric acid and concentrated, and hexane was added to cause crystallization. The crystal was filtered and dried to yield 2-(5-benzyloxycarbonylamino-6-oxo-2-phenyl-1,6-dihydropyrimidin-1-yl)acetic acid (210 mg, 0.56 mmol).
¹H-NMR(DMSO-d₆)δppm: 4.51(2H,s), 5.19(2H,s), 7.34-7.56(10H,m), 8.47(1H,s), 9.01(1H,s).

### Example 5

To 1-(tert-butoxycarbonylmethyl)-6-oxo-2-phenyl-5-benzoylamino-1,6-dihydropyrimidine (1.0 g, 2.5 mmol), methanol (10 ml) and sodium hydroxide (0.30 g, 7.5 mmol) were added, and the mixture was stirred under refluxing overnight. The mixture was cooled to 10°C, and the crystal was collected by filtration and washed with acetonitrile. The obtained crystal was dried to yield the sodium salt of 2-(5-amino-6-oxo-2-phenyl-1,6-dihydropyrimidin-1-yl)acetic acid (650 mg, 2.3 mmol).
¹H-NMR(DMSO-d₆)δppm: 4.09(2H,brs), 7.30(1H,s), 7.37-7.43(3H,m), 7.52-7.54(2H,m).
MS(ESI)M/Z MH+246.2

### Example 6

To 1-(tert-butoxycarbonylmethyl)-6-oxo-2-phenyl-5-benzoylamino-1,6-dihydropyrimidine (1.0 g, 2.5 mmol), methanol (8 ml) and lithium hydroxide monohydrate (0.41 g, 8 mmol) were added, and the mixture was stirred under refluxing overnight. The mixture was cooled to 10°C, and the crystal was collected by filtration and washed with acetonitrile. The obtained crystal was dried to yield the lithium salt of 2-(5-amino-6-oxo-2-phenyl-1,6-dihydropyrimidin-1-yl)acetic acid (560 mg, 2.2 mmol).

### Example 7

To 1-(tert-butoxycarbonylmethyl)-6-oxo-2-phenyl-5-benzoylamino-1,6-dihydropyrimidine (1.0 g, 2.5 mmol), methanol (8 ml) and sodium hydroxide (0.40 g, 10 mmol) were added, and the mixture was stirred under refluxing overnight. Water (5 ml) was added, the methanol was distilled off, methyl tert-butyl ether (3 ml) was added, and the mixture was adjusted to', pH 3 with 6N hydrochloric acid and stirred overnight. The precipitate was collected by filtration and washed with water and methyl tert-butyl ether, and the obtained crystal was dried to yield 2-(5-amino-6-oxo-2-phenyl-1,6-dihydropyrimidin-1-yl)acetic acid (585 mg, 2.4 mmol).
¹H-NMR(DMSO-d₆)δppm: 4.46(2H,s), 5.22(2H,brs), 7.34(1H,s), 7.41-7.49(5H,m).

### Example 8

The sodium salt of 2-(5-amino-6-oxo-2-phenyl-1,6-dihydropyrimidin-1-yl)acetic acid (560 mg, 2.1 mmol) was dissolved in water (6 ml), benzyloxycarbonyl chloride (0.43 ml, 2.5 mmol) was added gradually, next the solution was stirred at room temperature for 2 hours while adjusting to pH 6-8 with saturated aqueous sodium hydrogen carbonate (water: 3 ml). After the reaction solution was washed with toluene, ethyl acetate and hydrochloric acid were added to the organic layer, and the water layer was adjusted to pH 1 and extracted. After the organic layer was concentrated, hexane was added to cause crystallization. The crystal was filtered and dried to yield 2-(5-benzyloxycarbonylamino-6-oxo-2-phenyl-1,6-dihydropyrimidin-1-yl)acetic acid (610 mg, 1.6 mmol). The physical property values of the obtained compound were the same as Example 4.

### Example 9

To 1-(tert-butoxycarbonylmethyl)-6-oxo-2-phenyl-5-benzoylamino-1,6-dihydropyrimidine (0.5 g), trifluoroacetic acid (5 ml) was added, and the mixture was stirred at room temperature for 3 hours. The reaction solution was concentrated, and ethyl acetate was added to cause crystallization. The precipitate was collected by filtration and washed with ethyl acetate, and the obtained crystal was dried to yield 2-(5-benzoylamino-6-oxo-2-phenyl-1,6-dihydropyrimidin-1-yl)acetic acid (0.41 g).
¹H-NMR(DMSO-d₆)δppm: 4.56(2H,s), 7.53-7.65(8H,m), 7.97-8.00(2H,m), 8.80(1H,s), 9.57(1H,s).
MS(ESI)M/Z MH+350.2

### Example 10

Phenaceturic acid (3.0 g), triethyl ortho-formate (3.0 g), acetic anhydride (5.5 g) and toluene (10 ml) were stirred under refluxing for 3 hours. Subsequently, the reaction solution was cooled to room temperature, ethanol and toluene were added, and the mixture was concentrated to dryness. N-(tert-butoxycarbonylmethyl)phenylamidine hydrochloride (2.9 g, 10.8 mmol) was stirred in toluene (30 ml) and water, and sodium carbonate was added. After the organic layer was separated, the water layer was extracted by the addition of toluene (5 ml), and the organic layers were combined, washed with saline and dried with sodium sulfate. To the obtained solution, the previously obtained dry solid was added, and the mixture was stirred at 70°C overnight. The reaction solution was washed with 1N hydrochloric acid, saturated aqueous sodium hydrogen carbide and saturated saline, and the organic layer was concentrated to dryness to yield 1-(tert-butoxycarbonylmethyl)-6-oxo-2-phenyl-5-phenylacetylamino-1,6-dihydropyrimidine (1.7 g).
¹H-NMR(CDCl₃)δppm: 1.41(9H,s), 3.76(2H,s), 4.50(2H,s), 7.23-7.49(10H,m), 8.11(1H,s), 9.09(1H,s).
MS(ESI)M/Z MH+420.4

### Example 11

To 1-(tert-butoxycarbonylmethyl)-6-oxo-2-phenyl-5-benzoylamino-1,6-dihydropyrimidine (600 mg), methanol (5 ml) and 4N sodium hydroxide (3 ml) were added, and the mixture was stirred at 70°C for 3 hours. After the methanol was distilled off from the reaction solution, ethyl acetate was added, and the water layer was adjusted to pH 2 with hydrochloric acid and extracted. After the ethyl acetate layer was removed, the water layer was analyzed by liquid chromatography; 200 mg of 2-(5-amino-6-oxo-2-phenyl-1,6-dihydropyrimidin-1-yl)acetic acid was obtained.

### Example 12

To 1-(tert-butoxycarbonylmethyl)-6-oxo-2-phenyl-5-benzoylamino-1,6-dihydropyrimidine (300 mg), acetonitrile (1 ml) and 4N hydrochloric acid (3 ml) were added, and the mixture was stirred under refluxing overnight. The solution was extracted by the addition of ethyl acetate, and the organic layer was removed, after which the water layer was analyzed by liquid chromatography; 94 mg of 2-(5-amino-6-oxo-2-phenyl-1,6-dihydropyrimidin-1-yl)acetic acid was obtained.

### Example 13

To 1-(tert-butoxycarbonylmethyl)-6-oxo-2-phenyl-5-benzoylamino-1,6-dihydropyrimidine (150 mg), methanol (1.5 ml) and sodium hydroxide (100 mg) were added, and the mixture was stirred under refluxing overnight. After the methanol was distilled off from the reaction solution, water and ethyl acetate were added, the water layer was adjusted to pH 2 with hydrochloric acid and extracted, and the ethyl acetate layer was removed, after which the water layer was analyzed by liquid chromatography; 86 mg of 2-(5-amino-6-oxo-2-phenyl-1,6-dihydropyrimidin-1-yl)acetic acid was obtained.

### Example 14

To N-(tert-butoxycarbonylmethyl)methylamidine hydrochloride (200 mg, 1.0 mmol), toluene (4 ml), water (3 ml) and sodium carbonate (0.8 g) were added, the mixture was neutralized and extracted, and the toluene layer was washed with saturated saline. 4-ethoxymethylene-2-phenyl-5-azlactone (160 mg, 0.73 mmol) and acetonitrile (1 ml) was added, and the mixture was stirred at 80°C overnight. Subsequently, the solution was washed with 1N hydrochloric acid, saturated aqueous sodium bicarbonate and saturated saline, the organic layer was concentrated, and the precipitate was filtered and dried. 1-(tert-butoxycarbonylmethyl)-6-oxo-2-methyl-5-benzoylamino-1,6-dihydropyrimidine (130 mg) was obtained.
¹H-NMR(DMSO-d₆)δppm: 1.45(9H,s), 2.45(3H,s), 4.83(2H,s), 7.50-7.62(3H,m), 7.93-7.95(2H,m), 8.55(1H,s), 9.4(1H,s).
MS(ESI)M/Z MH+344.2

### Example 15

To N-(2,2-dimethoxyethyl)phenylamidine hydrochloride (1.5 g, 6.1 mmol), toluene (15 ml), water (10 ml) and sodium carbonate (2.5 g) were added, the mixture was neutralized and extracted, and the toluene layer was washed with saturated saline. 4-ethoxymethylene-2-phenyl-5-azlactone (0.67 g, 3.1 mmol) was added, and the mixture was stirred at 80°C overnight. Subsequently, the solution was washed with 1N aqueous hydrochloric acid, saturated aqueous sodium bicarbonate and saturated saline, and the organic layer was concentrated to dryness to yield 1-(2,2-dimethoxyethyl)-6-oxo-2-methyl-5-benzoylamino-1,6-dihydropyrimidine (1.0 g).
¹H-NMR(CDCl₃)δppm: 3.28(6H,s), 4.02(2H,d,J=5.8Hz), 4.76(1H,t,J=5.8Hz), 7.16-7.56(8H,m), 7.93-7.94(2H,m), 8.85(1H,brs), 9.25(1H,s).
MS(ESI)M/Z MH+380.1

To the 1-(2,2-dimethoxyethyl)-6-oxo-2-methyl-5-benzoylamino-1,6-dihydropyrimidine (500 mg, 1.3 mmol) obtained above, acetic acid (5 ml) was added, 3N hydrochloric acid (88 µl, 0.2 mmol) was added, and the mixture was stirred at 70°C for 4 hours, after which sodium acetate (32 mg, 0.4 mmol) was added, sodium perborate (405 mg, 2.6 mmol) was added drop by drop gradually, and the mixture was stirred at room temperature for 3 days. Subsequently, sodium thiosulfate (654 mg) was added, and the mixture was stirred for 1 hour. This was concentrated to dryness, water (10 ml) was added, the mixture was adjusted to pH 8 with sodium hydroxide and twice washed with toluene (5 ml). The water layer was adjusted to pH 2 with hydrochloric acid, and the precipitate was filtered, washed with water, and then dried to yield 2-(5-benzoylamino-6-oxo-2-phenyl-1,6-dihydropyrimidin-1-yl)acetic acid (0.33 g). The physical property values of the obtained compound were the same as Example 9.

### Example 16

To N-(tert-butoxycarbonyl)-4-fluorophenylamidine hydrochloride (230 mg, 0.8 mmol), toluene (4 ml), water (3 ml) and sodium carbonate (0.5 g) were added, the mixture was neutralized and extracted, and the toluene layer was washed with saturated saline. 4-ethoxymethylene-2-phenyl-5-azlactone (133 mg, 0.6 mmol) was added, and the mixture was stirred at 80°C overnight. Subsequently, the solution was washed with 1N aqueous hydrochloric acid, saturated aqueous sodium bicarbonate and saturated saline, and the organic layer was concentrated to dryness to yield 1-(tert-butoxycarbonyl)-6-oxo-2-(4-fluorophenyl)-5-benzoylamino-1,6-dihydropyrimidine (0.23 g).
¹H-NMR(CDCl₃)δppm: 1.49(9H,s), 4.64(2H,s), 7.15-7.57(6H,m), 7.94(2H,m), 8.80(1H,brs), 9.26(1H,s).
MS(ESI)M/Z MH+424.4

### Example 17

To N-carboxymethyl-phenylamidine sodium salt (100 mg, 0.4 mmol), acetonitrile (3 ml) and 4-ethoxymethylene-2-benzyl-5-azlactone (100 mg, 0.4 mmol) were added, and the mixture was stirred at 80°C overnight. Subsequently, the solution was concentrated and washed by the addition of water (3 ml) and ethyl acetate (3 ml), and the water layer was adjusted to pH 2 by the addition of hydrochloric acid and extracted by the addition of ethyl acetate. The organic layer was concentrated to dryness to yield 2-((5-phenylacetylamino)-6-oxo-2-phenyl-1,6-dihydropyrimidin-1-yl)acetic acid (122 mg).
¹H-NMR(DMSO-d₆)δppm: 3.84(2H,s), 4.52(2H,s), 7.29-7.55(10H,m), 8.83(1H,s), 9.71(1H,s).
MS(ESI)M/Z MH+364.2

### Example 18

To acetylglycine (0.4 g), triethyl ortho-formate (1.7 ml), acetic anhydride (3.2 ml), zinc chloride (47 mg) and sodium acetate (30 mg) were added, and the mixture was stirred at 80°C for 1.5 hours. The solution was analyzed by HPLC; 4-ethoxymethylene-2-methyl-5-azlactone was prepared with a yield of 52%. Toluene and ethanol was added, the mixture was concentrated to dryness, and the residue was purified by silica gel column chromatography (ethyl acetate-hexane) to yield 4-ethoxymethylene-2-methyl-5-azlactone (0.26 g).
¹H-NMR(CDCl₃)δppm: 1.45(3H,t,J=7.1Hz), 2.29(3H,s), 4.35(2H,q,J=7.1Hz), 7.21(1H,s).

### Example 19

To acetylglycine (1.0 g), trimethyl ortho-formate (1.36 g), acetic anhydride (3.1 g), zinc chloride (117 mg) and sodium acetate (86 mg) were added, and the mixture was stirred at 80°C for 1.5 hours. Toluene and ethanol were added, the mixture was concentrated to dryness, and the residue was purified by silica gel column chromatography (ethyl acetate-hexane) to yield 4-methoxymethylene-2-methyl-5-azlactone (0.54 g).
¹H-NMR(CDCl₃)δppm: 2.28(3H,s), 4.10(3H,s,), 7.13(1H,s).
MS(APCI)M/Z MH+142.1

### Example 20

To p-chlorobenzoylglycine (1.0 g), triethyl ortho-formate (0.97 g), acetic anhydride (1.9 g) and toluene (5 ml) were added, and the mixture was stirred at 100°C for 4 hours. Ethanol (0.5 g) and toluene (10 ml) were added, the mixture was concentrated, hexane was added, and the precipitate was collected by filtration to yield 4-ethoxymethylene-2-p-chlorophenyl-5-azlactone (0.82 g).
¹H-NMR(DMSO-d₆)δppm: 1.34(3H,t,J=7.1Hz), 4.46(2H,q,J=7.1Hz), 7.64(2H,d,J=2.5Hz), 7.79(1H,s,), 7.93(2H,d,J=2.5Hz).
MS(ESI)M/Z MH+251.94

### Example 21

To p-toloylglycine (1.0 g), triethyl ortho-formate (1.07 g), acetic anhydride (2.11 g) and toluene (1 ml) were added, and the mixture was stirred at 100°C for 4 hours. Ethanol (0.5 g) and toluene (10 ml) were added, the mixture was concentrated, hexane was added, and the precipitate was collected by filtration to yield 4-ethoxymethylene-2-p-tolyl-5-azlactone (0.75 g).
¹H-NMR(DMSO-d₆)δppm: 1.35(3H,t,J=7.1Hz), 2.39(3H,s), 4.46(2H,q,J=7.1Hz), 7.36(2H,d,J=8.1Hz), 7.73(1H,s,), 7.83(2H,d,J=8.1Hz).
MS(ESI)M/Z MH+231.91

### Example 22

To N-(tert-butoxycarbonylmethyl)phenylamidine hydrochloride (490 mg, 1.81 mmol), toluene (6 ml), water (3 ml) and sodium carbonate (0.8 g) were added, and the mixture was neutralized and extracted. The toluene layer was washed with saturated saline. 4-ethoxymethylene-2-p-chlorophenyl-5-azlactone (348 mg, 1.39 mmol) was added, and the mixture was stirred at 80°C overnight. Subsequently, the solution was washed with 1N aqueous hydrochloric acid, saturated aqueous sodium bicarbonate and saturated saline, the organic layer was concentrated, hexane was added to cause crystallization, and the precipitate was collected by filtration and dried. 1-(tert-butoxycarbonylmethyl)-6-oxo-2-phenyl-5-p-chlorobenzoylamino-1,6-dihydropyrimidine (584 mg, 1.33 mmol) was obtained.
¹H-NMR(CDCl₃)δppm: 1.46(9H,s), 4.58(2H,s), 7.47-7.54(7H,m), 7.87(2H,d,J=6.7Hz), 8.76(1H,s), 9.24(1H,s).
MS(ESI)M/Z MH+440.30,-438.30

### Example 23

To N-(tert-butoxycarbonylmethyl)phenylamidine hydrochloride (490 mg, 1.81 mmol), toluene (6 ml), water (3 ml) and sodium carbonate (0.8 g) were added, and the mixture was neutralized and extracted. The toluene layer was washed with saturated saline. 4-Ethoxymethylene-2-p-tolyl-5-azlactone (320 mg, 1.39 mmol) was added, and the mixture was stirred at 80°C overnight. Subsequently, the solution was washed with 1N aqueous hydrochloric acid, saturated aqueous sodium bicarbonate and saturated saline, the organic layer was concentrated, hexane was added to cause crystallization, and the precipitate was collected by filtration and dried. 1-(tert-Butoxycarbonylmethyl)-6-oxo-2-phenyl-5-p-toloylamino-1,6-dihydropyrimidine (550 mg, 1.31 mmol) was obtained.
¹H-NMR(CDCl₃)δppm: 1.46(9H,s), 2.43(3H,s), 4.58(2H,s), 7.29(2H,d,J=8.2Hz), 7.48-7.51(5H,m), 7.83(2H,d,J=8.2Hz), 8.78(1H,s), 9.27(1H,s).
MS(ESI)M/Z MH+420.37

### Example 24

To N-(carboxymethyl)phenylamidine (345 mg, 1.9 mmol), isopropyl alcohol (5 ml) and a 21% sodium ethoxide-ethanol solution (0.62 g) were added, and the mixture was stirred. Subsequently, 4-ethoxymethylene-2-p-chlorophenyl-5-azlactone (500 mg, 1.9 mmol) was added at 50°C, and the mixture was stirred at 80°C for 5 hours. Subsequently, the solution was concentrated and washed by the addition of water and ethyl acetate, the water layer was adjusted to pH 2 with an aqueous solution of hydrochloric acid. The precipitate was collected by filtration and dried to yield 2-(5-p-chlorobenzoylamino-6-oxo-2-phenyl-1,6-dihydropyrimidin-1-yl)acetic acid (401 mg, 1.1 mmol).
¹H-NMR(DMSO-d₆)δppm: 4.56(2H,s), 7.54-7.63(7H,m), 8.00(2H,d,J=4.7Hz), 8.76(1H,s), 9.72(1H,s).
MS(ESI)M/Z MH+384.1,MH-382.0

### Example 25

In formic acid (1.5 ml), acetic anhydride (0.5 ml) was added, and the mixture was stirred at room temperature for 1 hour, after which 2-(5-amino-6-oxo-2-phenyl-1,6-dihydropyrimidin-1-yl)acetic acid (100 mg, 0.41 mmol) was added, and the mixture was stirred at room temperature for 2 hours. Toluene (5 ml) was added, the mixture was concentrated and washed with ethyl acetate, and the precipitate was collected by filtration and dried under reduced pressure to yield 2-(5-formylamino-6-oxo-2-phenyl-1,6-dihydropyrimidinel-yl)acetic acid (108 mg, 0.40 mmol).
¹H-NMR(DMSO-d₆)δppm: 4.53(2H,s), 7.49-7.58(5H,m), 8.14(1H,s), 8.39(1H,s), 8.89(1H,s), 10.05(1H,s).
MS(ESI)M/Z MH-272.2

### Reference Example

To acetylglycine (0.4 g), triethyl ortho-formate (1.7 ml) and acetic anhydride (3.2 ml) were added, and the mixture was stirred at 80°C for 8 hours. The solution was analyzed by HPLC; 4-ethoxymethylene-2-methyl-5-azlactone was prepared with a yield of 22%.

### Industrial Applicability

According to the present invention, novel Pyrimidine Compound (1) can be prepared safely with a small number of steps at a relatively low cost, Acetic Acid Compound (6) can be prepared from Pyrimidine Compound (1) in good yield, and separation and purification of the obtained Acetic Acid Compound (6) can be conducted surprisingly easily. Furthermore, according to the present invention, N-Protected Compound (7) can also be prepared safely at a relatively low cost with a good yield. The Pyrimidine Compounds (1) and (1'), Acetic Acid Compound (6) and N-Protected Compounds (7) and (7') obtained by the methods of the present invention are useful as intermediates for pharmaceuticals such as enzyme inhibitors such as elastase inhibitors and chymase inhibitors.

This application is based on a patent application No. 174916/2002 filed in Japan, the contents of which are hereby incorporated by reference.

## Claims

1. A method of preparing a pyrimidine compound represented by the formula (1): 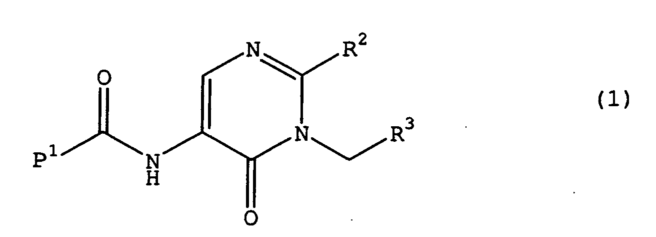 wherein P¹ represents a hydrogen atom, an alkyl group, an optionally substituted aryl group, an alkenyl group, an aralkyl group, a haloalkyl group or an optionally substituted amino group, R² represents an alkyl group having 1 to 4 carbon atoms or a phenyl group optionally substituted by at least one substituent selected from among an optionally substituted alkyl group, an alkoxy group, a nitro group, a hydroxy group, a halogen atom and an amino group and R³ represents a carboxyl group or a group that can be derivatized to a carboxyl group, provided that when P¹ is a phenyl group, then R³ should represent a group that can be derivatized to a carboxyl group, or a salt thereof, which comprises reacting an azlactone compound represented by the formula (2): 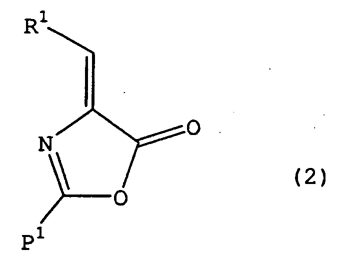 wherein R¹ represents an alkoxy group or a trialkylsiloxy group and P¹ is as defined above, or a salt thereof, with an amidine compound represented by the formula (3): 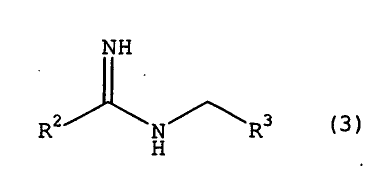 wherein R² is as defined above, or a salt thereof.

2. The production method of claim 1, wherein an acid adduct salt of the amidine compound represented by the formula (3) is neutralized with a base and thereafter reacted with the azlactone compound represented by the formula (2) or a salt thereof.

3. The production method of claim 1, wherein the azlactone compound represented by the formula (2) or a salt thereof obtained by reacting an α-aminocarboxylic acid represented by the formula (4): 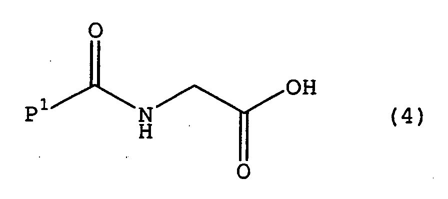 wherein P¹ is as defined in claim 1, or a salt thereof, with an ortho-formic acid ester represented by the formula (5): (R¹)₃CH wherein R¹ is as defined in claim 1, is used.

4. The production method of any of claims 1 to 3, wherein P¹ is an alkyl group, an optionally substituted aryl group, an alkenyl group, an aralkyl group, a haloalkyl group or an optionally substituted amino group.

5. The production method of any of claims 1 to 4, wherein P¹ is a chlorophenyl group, a tolyl group, a phenyl group, a benzyl group or a methyl group,
R¹ is a methoxy group or an ethoxy group,
R³ is a tert-butoxycarbonyl group or a carboxyl group, and
R² is a methyl group, a phenyl group or a fluorophenyl group.

6. The production method of any of claims 1 to 4, wherein P¹ is a phenyl group, a benzyl group or a methyl group, R¹ is a methoxy group or an ethoxy group, R³ is a tert-butoxycarbonyl group, and R² is a methyl group, a phenyl group or a fluorophenyl group.

7. A method of preparing an acetic acid compound represented by the formula (6) 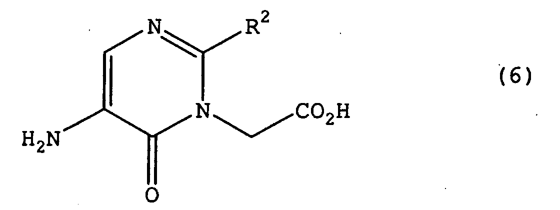 wherein R² represents an alkyl group having 1 to 4 carbon atoms or a phenyl group optionally substituted by at least one substituent selected from among an optionally substituted alkyl group, an alkoxy group, a nitro group, a hydroxy group, a halogen atom and an amino group, or a salt thereof, which comprises reacting an azlactone compound represented by the formula (2): 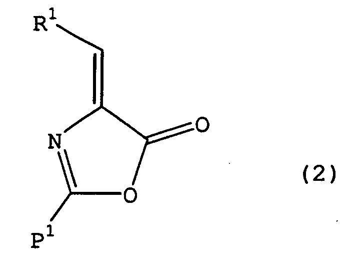 wherein P¹ is a hydrogen atom, an alkyl group, an optionally substituted aryl group, an alkenyl group, an aralkyl group, a haloalkyl group or an optionally substituted amino group and R¹ is an alkoxy group or a trialkylsiloxy group, or a salt thereof, with an amidine compound represented by the formula (3): 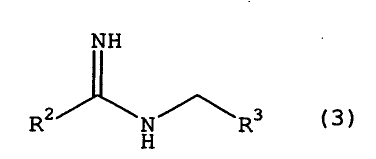 wherein R² is as defined above and R³ represents a carboxyl group or a group that can be derivatized to a carboxyl group, or a salt thereof, to give a pyrimidine compound represented by the formula (1) 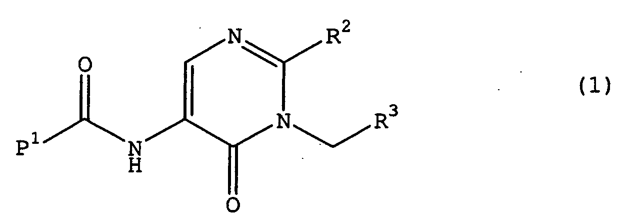 wherein P¹, R² and R³, are as defined above, or a salt thereof, and subjecting the obtained pyrimidine compound of the formula (1) or a salt thereof to a deprotection reaction, provided that when P¹ is a phenyl group, then R³ should represent a group that can be derivatized to a carboxyl group.

8. The production method of claim 7, wherein P¹ is an alkyl group, an optionally substituted aryl group, an alkenyl group, an aralkyl group, a haloalkyl group or an optionally substituted amino group.

9. A method of preparing an acetic acid compound represented by the formula (6) 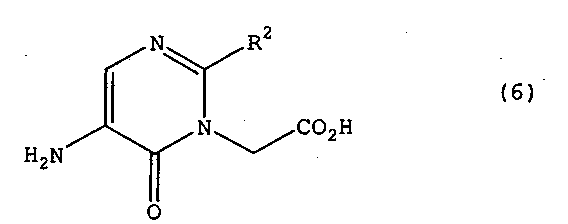 wherein R² represents an alkyl group having 1 to 4 carbon atoms or a phenyl group optionally substituted by at least one ', substituent selected from among an optionally substituted alkyl group, an alkoxy group, a nitro group, a hydroxy group, a halogen atom and an amino group, or a salt thereof, which comprises subjecting a pyrimidine compound represented by the formula (1) 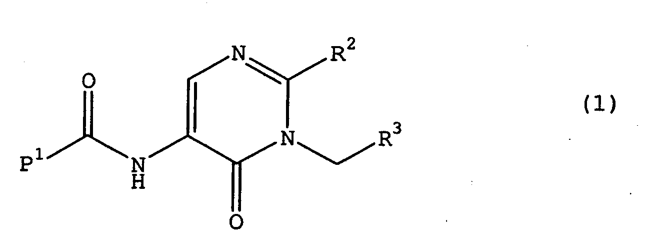 wherein P¹ represents a hydrogen atom, an alkyl group, an optionally substituted aryl group, an alkenyl group, an aralkyl group, a haloalkyl group or an optionally substituted amino group, R² is as defined above and R³ represents a carboxyl group or a group that can be derivatized to a carboxyl group, or a salt thereof, to a deprotection reaction, wherein when P¹ is a phenyl group, then R³ should be a group that can be derivatized to a carboxyl group.

10. The production method of claim 9, wherein P¹ is an alkyl group, an optionally substituted aryl group, an alkenyl group, an aralkyl group, a haloalkyl group or an optionally substituted amino group.

11. The production method of claim 7, 9 or 10, wherein a pyrimidine compound represented by the formula (1) or a salt thereof is subjected to a deprotection reaction in an alcohol in the presence of an alkali metal hydroxide.

12. The production method of claim 10, wherein P¹ is a phenyl group.

13. A method of preparing an N-protected compound represented by the formula (7): 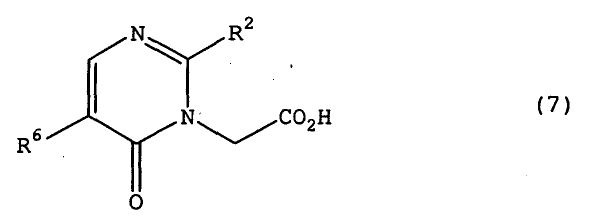 wherein R² represents an alkyl group having 1-4 carbon atoms or a phenyl group optionally substituted by at least one substituent selected from among an optionally substituted alkyl group, an alkoxy group, a nitro group, a hydroxy group, a halogen atom and an amino group, and R⁶ represents an -NR⁵H group or an -N(R⁵)₂ group wherein R⁵ represents an amino-protecting group, or a salt thereof, which comprises the following steps 1 to 3;
step 1: reacting an azlactone compound represented by the formula (2) 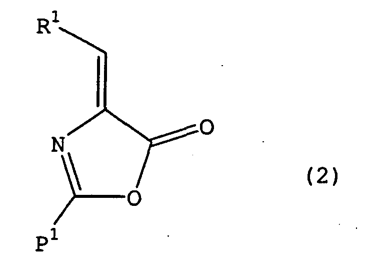 wherein P¹ represents a hydrogen atom, an alkyl group, an optionally substituted aryl group, an alkenyl group, an aralkyl group, a haloalkyl group or an optionally substituted amino group and R¹ represents an alkoxy group or a trialkylsiloxy group, or a salt thereof, with an amidine compound represented by the formula (3): 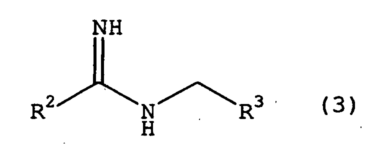 wherein R² is as defined above and R³ represents a carboxyl group or a group that can be derivatized to a carboxyl group, or a salt thereof, to yield a pyrimidine compound represented by the formula (1) 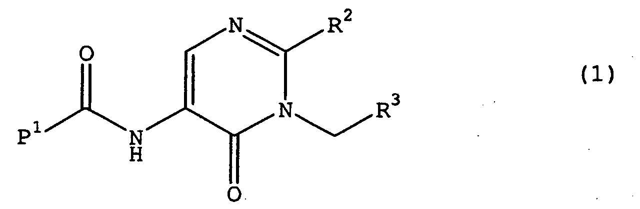 wherein P¹, R² and R³ are as defined above, or a salt thereof, step 2: subjecting the obtained pyrimidine compound of the formula (1) or a salt thereof to a deprotection reaction to yield an acetic acid compound represented by the formula (6) 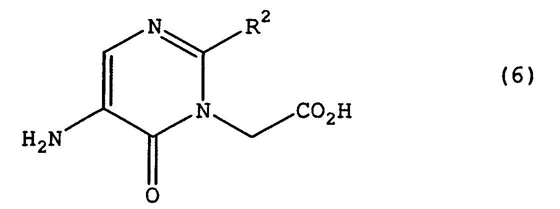 wherein R² is as defined above, or a salt thereof,
step 3: subjecting the obtained acetic acid compound of the formula (6) or a salt thereof to a reaction for protecting the amino group substituted at the 5-position of the pyrimidine ring to yield the N-protected compound of the above formula (7) or a salt thereof;
provided that when P¹ is a phenyl group, then R³ should be a group that can be derivatized to a carboxyl group.

14. The production method of claim 13, wherein P¹ is an alkyl group, an optionally substituted aryl group, an alkenyl group, an aralkyl group, a haloalkyl group or an optionally substituted amino group.

15. The production method of claim 13 or 14, wherein step 2 is conducted in an alcohol in the presence of an alkali metal hydroxide.

16. A method of preparing an N-protected compound represented by the formula (7'): 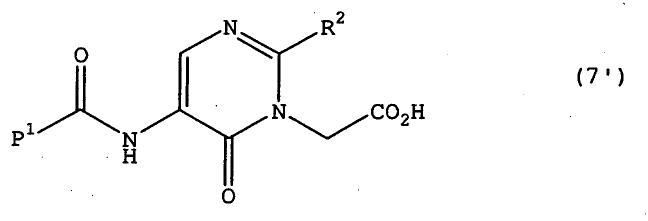 wherein P¹ represents a hydrogen atom, an alkyl group, an optionally substituted aryl group, an alkenyl group, an aralkyl group, a haloalkyl group or an optionally substituted amino group, and R² represents an alkyl group having 1 to 4 carbon atoms or a phenyl group optionally substituted by at least one substituent selected from among an optionally substituted alkyl group, an alkoxy group, a nitro group, a hydroxy group, a halogen atom and an amino group, or a salt thereof, which comprises derivatizing R³' in a pyrimidine compound represented by the formula (1'): 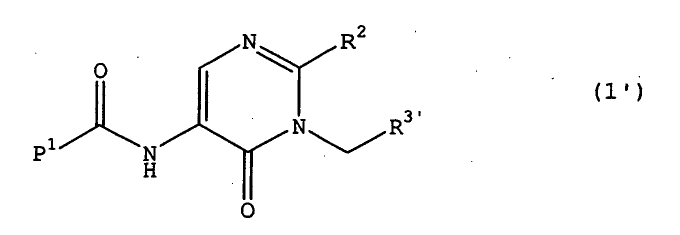 wherein P¹ and R² are as defined above and R³' represents a group that can be derivatized to a carboxyl group, or a salt thereof, to a carboxyl group.

17. The production method of claim 16, wherein P¹ is an alkyl group, an optionally substituted aryl group, an alkenyl group, an aralkyl group, a haloalkyl group or an optionally substituted amino group.

18. A pyrimidine compound represented by the formula (1) 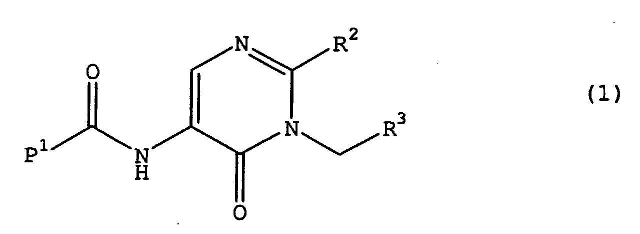 wherein P¹ represents a hydrogen atom, an alkyl group, an optionally substituted aryl group, an alkenyl group, an aralkyl group, a haloalkyl group or an optionally substituted amino group, R² represents an alkyl group having 1 to 4 carbon atoms or a phenyl group optionally substituted by at least one substituent selected from among an optionally substituted alkyl group, an alkoxy group, a nitro group, a hydroxy group, a halogen atom and an amino group and R³ represents a carboxyl group or a group that can be derivatized to a carboxyl group, provided that when P¹ is a phenyl group, then R³ should represent a group that can be derivatized to a carboxyl group, or a salt thereof.

19. The pyrimidine compound of claim 18, wherein P¹ is an alkyl group, an optionally substituted aryl group, an alkenyl group, an aralkyl group, a haloalkyl group or an optionally substituted amino group, or a salt thereof.

20. The pyrimidine compound of claim 19, wherein P¹ represents an alkyl group, an aryl group or an aralkyl group, R² represents a phenyl group or an alkyl group having 1-4 carbon atoms, which is optionally substituted by a halogen atom, and R³ represents a carboxyl group or a group that can be derivatized to a carboxyl group.

21. A method of preparing an azlactone compound represented by the formula (2) 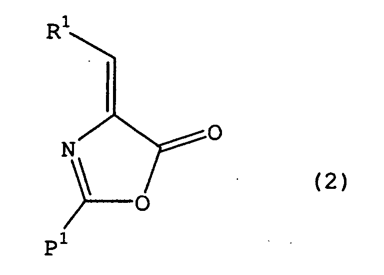 wherein P¹ represents a hydrogen atom, an alkyl group, an optionally substituted aryl group, an alkenyl group, an aralkyl group, a haloalkyl group or an optionally substituted amino group and R¹ represents an alkoxy group or a trialkylsiloxy group, or a salt thereof, which comprises reacting an α-aminocarboxylic acid represented by the formula (4) 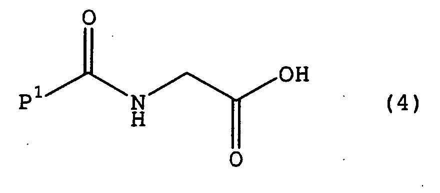 wherein P¹ is as defined above, or a salt thereof, with an ortho-formic acid ester represented by the formula (5): (R¹)₃CH wherein R¹ is as defined above, in the presence of acetic anhydride, zinc chloride and sodium acetate.

22. An azlactone compound represented by the formula (2'): 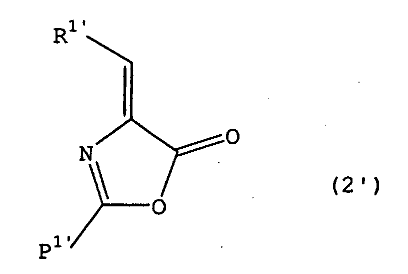 wherein P¹' represents an alkyl group, and R¹' represents an alkoxy group.

23. The azlactone compound of claim 22, wherein P¹' is a methyl group, an ethyl group, an isopropyl group, a tert-butyl group or an n-propyl group, and R¹' is a methoxy group or an ethoxy group.

24. A method of preparing the azlactone compound of claim 22 or a salt thereof, which comprises reacting an α-aminocarboxylic acid represented by the formula (4'): 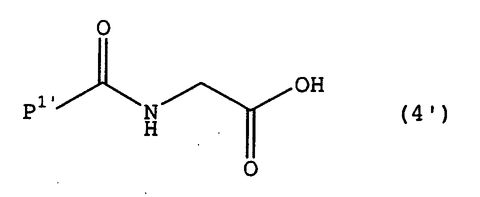 wherein P¹' represents an alkyl group, or a salt thereof with an ortho-formic acid ester represented by the formula (5'): (R¹')₃CH wherein R¹' represents an alkoxy group.
